(19)

**Europäisches Patentamt**
**European Patent Office**
**Office européen des brevets**

(11) **EP 2 669 677 B1**

(12) **EUROPÄISCHE PATENTSCHRIFT**

(45) Veröffentlichungstag und Bekanntmachung des Hinweises auf die Patenterteilung:
**12.09.2018 Patentblatt 2018/37**

(51) Int Cl.:
*G01N 33/49* *(2006.01)*     *B01F 11/00* *(2006.01)*

(21) Anmeldenummer: **12170284.9**

(22) Anmeldetag: **31.05.2012**

(54) **Verfahren und Vorrichtung zur Beschleunigung der Äquilibrierung einer Flüssigkeit**

Device and procedure for accelerating the equilibration of a liquid

Procédé et dispositif d'accélération de l'équilibrage d'un liquide

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**04.12.2013 Patentblatt 2013/49**

(73) Patentinhaber:
• **F. Hoffmann-La Roche AG**
  **4070 Basel (CH)**
  Benannte Vertragsstaaten:
  **AL AT BE BG CH CY CZ DK EE ES FI FR GB GR HR HU IE IS IT LI LT LU LV MC MK MT NL NO PL PT RO RS SE SI SK SM TR**
• **Roche Diagnostics GmbH**
  **68305 Mannheim (DE)**
  Benannte Vertragsstaaten:
  **DE**

(72) Erfinder:
• **Pfeiffer, Christoph**
  **2544 Leobersdorf (AT)**
• **Hofmann, Wolfgang**
  **8055 Graz (AT)**
• **Rüther, Horst**
  **8047 Hart/Graz (AT)**

(74) Vertreter: **Babeluk, Michael**
  **Patentanwalt**
  **Florianigasse 26/3**
  **1080 Wien (AT)**

(56) Entgegenhaltungen:
WO-A1-2010/018394     US-A- 4 871 439
US-A- 5 023 186     US-A1- 2011 065 084

• JACEM KILANI ET AL: "Study of the oxygen transfer in a disposable flexible bioreactor with surface aeration in vibrated medium", APPLIED MICROBIOLOGY AND BIOTECHNOLOGY, SPRINGER, BERLIN, DE, Bd. 74, Nr. 2, 30. November 2006 (2006-11-30), Seiten 324-330, XP019489485, ISSN: 1432-0614

**Beschreibung**

Die Erfindung betrifft ein Verfahren und eine Vorrichtung zur Beschleunigung der Äquilibrierung einer Flüssigkeit, vorzugsweise einer Referenzflüssigkeit eines Analysators, deren Flüssigkeitsoberfläche mit einer Gasphase in Kontakt steht, mit einer in einen Analysator austauschbar einsetzbaren Kassette, die zumindest einen flexiblen, gasdichten Beutel aufnimmt, in welchem die Flüssigkeit und die Gasphase aufgenommen sind.

Bei der Referenzflüssigkeit handelt es sich um eine Flüssigkeit, die für die Qualitätskontrolle und zur Kalibrierung von Analysatoren, beispielsweise zur Bestimmung der Parameter $pO_2$, $pCO_2$, pH und ggf. weiterer Parameter von Blut-Gas-Messgeräten (Blutgasanalysatoren), verwendet werden kann, wobei die Flüssigkeit zusammen mit einer Gasphase in einem flexiblen, gasdichten Behälter eingeschlossen ist.

Unter Äquilibrierung bzw. Gleichgewichtseinstellung einer Flüssigkeit ist in diesem Zusammenhang die Anpassung der Konzentrationen $c_i$ der in der Flüssigkeit gelösten Gase an die jeweiligen Partialdrucke $p_i$ der Gase der Gasphase, nach dem Henry'schen Gesetz

$$c_i = \alpha_i * p_i$$

zu verstehen, wobei $c_i$ die Konzentration der $i^{ten}$ Gaskomponente in der Flüssigkeit, $p_i$ der Partialdruck der $i^{ten}$ Gaskomponente in der Gasphase und $\alpha_i$ die temperaturabhängige Henry-Konstante der $i^{ten}$ Gaskomponente bedeuten. Im Unterschied zu den gelösten Gasen ergibt sich der temperaturabhängige Partialdruck der Flüssigkeit (z.B. $H_2O$) in der Gasphase durch die Clausius-Clapeyron-Gleichung. Bei einer Änderung der Partialdrücke $p_i$ und/oder der Temperatur stellt sich ein neues Gleichgewicht ein. Der im Gasraum des verschlossenen flexiblen Behälter vorliegende Gesamtdruck $p_{Gesamt}$ ist gleich dem Außendruck, d.h., dem barometrischen Luftdruck: $p_{Gesamt} = p_{Baro}$. Der Gesamtdruck der Gasphase ist wiederum gleich der Summe der Partialdrücke der einzelnen in der Gasphase befindlichen Gase (z.B. $N_2$, $O_2$, $CO_2$, $H_2O$):

$$p_{Gesamt} = \sum p_i$$

Somit ist die Lage des Gleichgewichts und damit die Verteilung der einzelnen Gaskomponenten (z.B. $O_2$, $N_2$, $CO_2$, $H_2O$) zwischen Gasphase und Flüssigkeit abhängig vom barometrischen Druck und der Temperatur. Dies gilt insbesondere für $O_2$ und $N_2$. $CO_2$ reagiert in der wässrigen Lösung zu $H_2CO_3$ und weiter zu $HCO_3^-$, $CO_3^{2-}$ und $H^+$, so dass hier noch weitere chemische Gleichgewichte eine Rolle spielen. Über die thermodynamischen Gleichgewichte des gelösten $CO_2$ mit $HCO_3^-$, $CO_3^{2-}$ und $H^+$ ist indirekt auch der pH-Wert der Flüssigkeit abhängig vom $pCO_2$ der Gasphase.

Ist der flexible Behälter und die darin enthaltene Flüssigkeit in Ruhe, so ist die Kinetik der Gleichgewichtseinstellung abhängig von der Kinetik des Übertritts der einzelnen Gaskomponente vom Gasraum in die Flüssigkeit und vice versa, sowie von der Diffusionsgeschwindigkeit der einzelnen Gaskomponenten innerhalb der Flüssigkeit. Letzteres gilt vor allem für Gase, die unter Normalbedingungen nicht mit der Flüssigkeit reagieren (z.B. $O_2$ und $N_2$). Bei $CO_2$ sind darüber hinaus Reaktionen mit der wässrigen Flüssigkeit und den darin enthaltenen pH-relevanten Substanzen zu berücksichtigen.

Das thermodynamische Gleichgewicht ist dann erreicht, wenn die Verteilung jeder einzelnen Gaskomponente zwischen Gasphase und Flüssigkeit dem Henry'schen Gesetz entspricht und die Konzentration jeder einzelnen Gaskomponente bzw. deren Reaktionsprodukte im gesamten Flüssigkeitsvolumen gleichmäßig verteilt vorliegt. Eine Änderung des barometrischen Drucks, beispielsweise durch die Wetterlage, führt zu einer Änderung des Gesamtdrucks $p_{Gesamt}$ der Gasphase im flexiblen Behälter und damit der Partialdrücke $p_i$ der einzelnen Gase und infolge dessen zu einer Verschiebung der Gleichgewichtslage. Bedingt durch die Temperaturabhängigkeit der Henry'schen Konstante und des Gaspartialdrucks der Flüssigkeit gilt ähnliches für Temperaturänderungen. Nach jeder Änderung dieser Art findet zur Einstellung des neuen Gleichgewichts ein Massentransport (Stofftransport) statt. Die Zeit, die benötigt wird, bis der neue Gleichgewichtszustand erreicht wird, hängt von den geometrischen Verhältnissen im flexiblen Behälter und dem jeweiligen Verhältnis von Flüssigkeits- und Gasphase ab. Diese ändern sich insbesondere mit zunehmendem Verbrauch der Flüssigkeit.

Wesentliche Einflussgrößen auf die Kinetik der Gleichgewichtseinstellung sind die Fläche der Flüssigkeitsoberfläche zur Gasphase und die Länge der Diffusionsstrecken der Gaskomponenten bzw. deren Reaktionsprodukte im Flüssigkeitsvolumen.

DEFINITIONEN:

Analyt

Der Analyt oder die Analyte sind die im Probenmedium - beispielsweise Blut, Blutplasma, Blutserum oder eine sonstige Körperflüssigkeit - enthaltenen Stoffe, über deren Vorhandensein und/oder Konzentration bei der Analyse (Messung) des Probenmediums mit einem Analysator eine Aussage getroffen werden soll. Analyte sind beispielsweise $O_2$, $CO_2$ und der pH-Wert.

Analysator bzw. pH/Blutgasanalysator

In einer einfachen Ausführung misst ein (automatischer) pH/Blutgas-Analysator den Sauerstoffpartialdruck ($pO_2$), den Partialdruck des Kohlendioxids ($pCO_2$), den pH-Wert und gegebenenfalls den barometrischen Druck mit Hilfe von Sensoren. Die komplette Messung dauert bei einem Probevolumen von 50-100 $\mu$l Vollblut in der Regel ca. 30-60 Sekunden. Manche pH/Blutgasanalysatoren messen als zusätzliche Analyten auch die Blutelektrolyte (Na$^+$, K$^+$ Ca$^{++}$, Cl$^-$), gewisse Metabolite (Glucose, Lactat, Harnstoff und Kreatinin) und die Hämoglobinwerte und berechnen weitere, davon abgeleitete Parameter.

Sensoren

Die Messungen erfolgen allgemein in austauschbaren Messkammern, die mit elektrochemischen (Elektroden) und/oder optischen (Optoden) sensorischen Elementen ausgestattet sind. Weiters werden hier auch photometrische/spektroskopische Methoden eingesetzt, wobei die optischen Eigenschaften der zu bestimmenden Probe oder Farbreaktionen zum Nachweis verwendet werden.

Äquilibrierung (Gleichgewichtseinstellung)

*Synonyme/engl.: equilibration*

Unter Äquilibrierung wird hier die Einstellung des Gleichgewichts zwischen einer Flüssigkeit und einer damit in direktem Kontakt stehenden Gasphase verstanden, wobei die Gasphase mehrere Gase enthalten kann. Im Gleichgewicht ist der Nettomassentransport zwischen Gasphase und Flüssigkeit Null, und die einzelnen Gaskomponenten sowie deren allfällige Reaktionsprodukte sind in der Flüssigkeitsphase im Wesentlichen gleichmäßig verteilt.

Kalibrierflüssigkeit

*Synonyme/engl.: Kalibrierflüssigkeit, Kalibriermedium, calibration liquid, reference liquid/solution, reference material*

Es handelt sich hierbei um wässrige Flüssigkeiten mit bekannten, unterschiedlichen, im Erwartungsbereich der Messgröße verteilten Konzentrationen eines oder mehrerer Analyte. Kalibrierflüssigkeiten werden zur Kalibration der einzelnen Sensoren verwendet. Kalibrierflüssigkeiten sind beispielsweise aus der US 4,116,336 (Radiometer) bekannt.

Kontrollflüssigkeit

*Synonyme/engl.:Kontrollflüssigkeit, QC-Flüssigkeit, QC-liquid, control liquid/- solution*

Es handelt sich hierbei - ebenso wie bei den Kalibrierflüssigkeiten - um wässrige Flüssigkeiten mit bekannten, unterschiedlichen, im Erwartungsbereich der Messgröße verteilten Konzentrationen eines oder mehrerer Analyte. Kontrollflüssigkeiten werden zur Überprüfung des Meßsystems und/oder einzelner Sensoren verwendet. Derartige Flüssigkeiten sind beispielsweise aus der US 4,116,336 (Radiometer) bekannt.

Referenzflüssigkeit

Im Folgenden werden Kalibrier- und Kontrollflüssigkeiten unter dem Begriff Referenzflüssigkeit zusammengefasst, insofern auf beide Prozesse (Kalibration und Kontrolle) Bezug genommen wird. Kalibrier- und Kontrollflüssigkeiten können im Wesentlichen ident zusammengesetzt sein. Ihre Anwendung ist jedoch eine unterschiedliche. Aus technischen und regulatorischen Gründen macht es keinen Sinn, eine Kalibration und anschließend eine Kontrolle des so kalibrierten Sensors mit ein und derselben Flüssigkeit durchzuführen. Bei dieser Vorgangsweise können in der Regel allfällige Fehler (z.B. fehlerhafte Flüssigkeitszusammensetzung, falsche Parameterwerte, Sensorfehler, Systemfehler, etc.) nicht erkannt werden.

Kalibration

*Synonyme/engl.: calibration; referencing*

Die (anwenderseitige) Kalibration ist das Verfahren zur Ermittlung der Kennlinie der einzelnen Sensoren in Form von Kennlinienparametern. Bei der Kalibration eines Sensors wird dieser mit verschiedenen Kalibriermedien in Kontakt gebracht, welche den Analyten in unterschiedlichen bekannten Konzentrationen enthalten.

Im Wesentlichen wird zur Ermittlung der Kennlinie das Signal des Sensors mit einer Reihe von wässrigen Kalibriermedien bekannter, unterschiedlicher, im Erwartungsbereich der Messgröße verteilten Konzentrationen des zu bestimmenden Analyten gemessen. Mit Hilfe der gewonnenen Kalibrierwerte werden die Parameter der Kennlinie bestimmt (beispielsweise Offset, Steigung, Signalhub, etc.).

Kontrollmessung

*Synonyme/engl.: QC, quality control,*

Bei der Kontrollmessung, genauer Qualitätskontrolle (QC-Messung) werden die Sensoren des Analysators mit einer oder mehreren Kontrollflüssigkeiten in Kontakt gebracht, welche den/die Analyte mit bekannten Istwerten enthalten. Mit den bei der Kontrollmessung erhobenen Istwerten, wird die Performance der Sensoren und des Systems überwacht. Dabei werden Soll- und Istwerte verglichen, wonach in Abhängigkeit der Differenzwerte bestimmte Folgemaßnahmen (corrective actions) ergriffen werden, beispielsweise Freigabe der Probemessung, Kalibration, Überprüfung des Systems, Ersetzen von Komponenten etc.

Die Kalibration von Blutgasanalysatoren bzw. deren Sensoren erfolgt in vorbestimmten Zeitintervallen mit Kalibrierflüssigkeiten, die in austauschbaren Gebinden im Analysator vorliegen. Im Allgemeinen erlaubt die in diesen Gebinden vorliegende Flüssigkeitsmenge eine Vielzahl an Kalibriermessungen.

Qualitätskontrollen für Blutgasanalysatoren sehen die regelmäßige Messung von Kontrollflüssigkeiten vor. Durch Vergleich der aktuell erfassten Messwerte für die Analyte $O_2$, $CO_2$ und pH und ggf. weiterer Analyte mit den Zielwerten der Kontrollflüssigkeiten wird die Qualität der Sensoren und des Messsystems bewertet. Im Allgemeinen wird für jede Qualitätskontrollmessung eine Ampulle mit Kontrollflüssigkeit verbraucht. Qualitätskontrollmessungen sind dementsprechend kostenintensiv. Der Einsatz von Gebinden, aus denen mehrfach Kontrollflüssigkeit angesaugt und vermessen wird, ist daher wünschenswert.

Obwohl bei der Kalibration bzw. Kontrollmessung einzig die Analytwerte der Kalibrier- bzw. Kontrollflüssigkeiten relevant sind, kommt dem Konzept der Bereitstellung dieser Flüssigkeiten entscheidende Bedeutung zu. Dies gilt im besonderen Maße für die Analytwerte in der Blutgasanalyse, da diesen die Konzentrationen von in der Flüssigkeit gelösten Gasen ($CO_2$, $O_2$) zugrunde liegen. Wie oben beschrieben, unterliegen die in der Flüssigkeit gelösten Gase einem Austausch mit der in Kontakt stehenden Gasphase gemäß dem Henry'schen Gesetz. Eine in nicht gasdicht verschlossenen und damit mit der Atmosphäre in Kontakt stehenden Gebinden bereitgestellte Referenzflüssigkeit verändert daher die Analytwerte für die Messparameter unausweichlich mit der Zeit.

Zur Überwindung dieser Problematik offenbart die US 3,681,255 (Wilfore) und die US 4,871,439 (Enzer) Konzepte mit "Headspace-freien" Beuteln zur Aufbewahrung von Kalibrier- und Kontrollflüssigkeiten. Dabei werden die Flüssigkeiten in gasdichten flexiblen Beuteln ohne Gasphase und unter Vermeidung von Gasblasen abgefüllt. Ein Austausch von in der Flüssigkeit gelösten Gasen mit einer Gasphase ist damit unterbunden. Auch die mehrfache Entnahme von großen Flüssigkeitsmengen über die Zeit im Betrieb stellt kein Problem dar, da die Beutel über Schläuche mit der Fluidik des Analysators verbunden sind und die sukzessive Flüssigkeitsentnahme lediglich eine Volumenverkleinerung der flexiblen Beutel bewirkt.

Nachteilig wirkt sich bei diesem Konzept insbesondere die ungenügende Stabilisierung von Sauerstoff aus. Da dieser nur in geringer Menge in der wässrigen Flüssigkeit gelöst ist, bewirkt eine geringfügige Abnahme von Sauerstoff in der Flüssigkeit eine starke Änderung des Analytwertes für den Blutgasparameter $pO_2$, so dass ein über die Lagerung des Gebindes stabiler bzw. voraussagbarer Zielwert für diesen Parameter nicht gewährleistet werden kann. Der Abbau geringer Sauerstoffmengen in der Lösung erfolgt beispielsweise durch Reaktion von Sauerstoff entweder mit dem Folienmaterial oder aber durch weitere in der Flüssigkeit enthaltene Substanzen, mit denen der Sauerstoff über die Zeit reagiert. Eine weitere Quelle für Änderungen des Sauerstoffgehalts ist die langsame Diffusion durch Nahtstellen des Gebindes.

Konzepte zur Stabilisierung von Sauerstoff in Headspace-freien Beuteln sind in der US 4,116,336 (Radiometer), US 5,780,302 (Chiron), US 7,378,006 (Instrumentation Laboratory) beschrieben.

Die US 5,780,302 beschreibt eine Methode zur Stabilisierung des $O_2$ durch Verpacken der Lösung in einem Gebinde bestehend aus einem Laminat. Das Laminat besteht aus a) einer inneren Schicht (head sealable polymer consisting polypropylene), b) einer mittleren Schicht (aluminum), c) einer äußeren Schicht (polyester nylon and a lacquer coating) und d) einem access device welches sich komplett innerhalb des Gebindes befindet.

Die US 7,378,006 offenbart zur Stabilisierung der $pO_2$- Werte den Zusatz von Cholin zur Flüssigkeit.

Die US 6,405,872 (Roche) schlägt für die Mehrfachentnahme tonometrierter, mit Gas äquilibrierter Flüssigkeiten gasdichte flexible Behälter mit einer Gasphase als Gasreservoir zur Stabilisierung der Gaswerte vor. Dabei ist das Gebinde mit einer Leitung an die Fluidik des Analysators angeschlossen. Nachteilig bei diesem Konzept ist jedoch, dass abhängig von der Größe des Gebindes und bedingt durch die oben beschriebenen physikalischen Gegebenheiten die (erneute) Gleichgewichtseinstellung nach raschen Temperatur- und Druckänderungen nur langsam über mehrere Stunden erfolgt.

Ein Konzept zur Stabilisierung von Sauerstoff in Kontrollflüssigkeiten, die in Glasampullen vorliegen, stellt der Kontakt der Flüssigkeit zu einer Gasphase innerhalb der Glasampulle dar. Die große Menge an Sauerstoff in der Gasphase stabilisiert den im Vergleich zur Gasphase deutlich niedrigeren und damit empfindlichen Sauerstoffgehalt einer rein wässrigen oder salzhaltigen Kontrollflüssigkeit. Ein Nachteil einer solchen Gasphase ist, dass aufgrund der Temperaturabhängigkeit der Henry-Konstante eine Temperatursensitivität des Sauerstoffgehalts der Lösung in Kauf genommen werden muss. Die Gleichgewichtsverteilung der einzelnen Komponenten zwischen Gasphase und Lösung bei verschiedenen Temperaturen sind bei kleinen Volumina in Glasampullen berechenbar und auch experimentell zugänglich. Aufgrund der Gleichgewichtsverteilung können Zielwerte für die Blutgasparameter vorausgesagt werden. In den bekannten Ampullen liegen etwa gleiche Volumina an Flüssigkeit und Gas vor und es existiert eine, bezogen auf das Volumen der Flüssigkeit relativ große Phasengrenzfläche, die zudem aufgrund der Starrheit der aufrecht stehenden Glasampulle immer konstant ist. Auch bei sich ändernder Temperatur ist damit eine ausreichende Voraussagbarkeit der effektiven Gaswerte gegeben. Der Einsatz von Ampullen hat den wirtschaftlichen Nachteil, dass für jede QC- oder Kontroll-Messung eigens eine Ampulle verbraucht wird, da sich nach dem Öffnen die Gaswerte durch Gasaustausch mit der umgebenden Atmosphäre rasch verstellen.

Im Gegensatz zu den Verhältnissen bei der oben erwähnten kleineren, starren Ampulle mit ca. 1-2 ml Inhalt, stellt sich das Verteilungsgleichgewicht bei größeren Flüssigkeitsmengen erst nach mehreren Stunden ein. Bei einer Flüssigkeitsmenge von 150 bis 300 ml und einer Oberfläche von 15 cm$^2$ (und somit einer Höhe der Flüssigkeitssäule von 10-20 cm) liegt die Äquilibrierungszeit oder auch Angleichzeit, also die Zeit, die infolge einer relativ schnellen Temperatur- oder Druckveränderung notwendig ist, die Gaskonzentrationen innerhalb der Flüssigkeit mit genügender Genauigkeit homogen (verteilt) einzustellen, bei ca. 36 Stunden. Insbesondere nach dem Einsetzen eines neuen Gebindes mit Referenzflüssigkeiten in den Analysator oder nach längeren Standzeiten des Analysators bei unklaren Temperaturverhältnissen, kommt es daher zu Verzögerungen der Betriebsfähigkeit oder zu Messfehlern.

Für Kalibrier- und Kontrollflüssigkeiten (Referenzflüssigkeiten) bieten sich im Analysator austauschbare Kassetten mit einem oder mehreren gasdichten flexiblen Behältern, z.B. Beutel aus gasundurchlässigem Folienmaterial an.

Hierbei ist es notwendig, den in der Flüssigkeit gelösten Sauerstoff, der als Vorgabewert bzw. Analyt für den in der Kalibrier- bzw. Kontrollflüssigkeit vorhandenen Parameter Sauerstoffpartialdruck (pO$_2$) dient, zu stabilisieren.

Dafür bietet sich, wie oben erwähnt, der Einschluss eines Gasraumes (head space) im Beutel an. Die große Menge an Sauerstoff in der Gasphase stabilisiert den im Vergleich zur Gasphase deutlich niedrigeren und damit empfindlichen Sauerstoffgehalt einer rein wässrigen oder salzhaltigen Kontrollflüssigkeit, allerdings muss aufgrund der Temperaturabhängigkeit der Henry-Konstante eine Temperatursensitivität des Sauerstoffgehalts der Lösung in Kauf genommen werden. Dem steht jedoch entgegen, dass der Gasaustausch zwischen wässriger Lösung und Gasphase rein diffusionsvermittelt ist, und sich, im Gegensatz zu den Verhältnissen in der oben erwähnten kleineren Ampulle, die Gleichgewichtszustände erst nach mehreren Stunden einstellen.

Flexible Behälter sind beispielsweise die in der US 6,405,872 beschriebenen Beutel. Diese bestehen aus einem mehrlagigen, gasdichten Folienmaterial, das an den Rändern verschweißt ist. Bisherige Erfahrungen haben gezeigt, dass beispielsweise mit einem handelsüblichen einfachen Aluminium-Laminat mit Polyethylen-Beschichtung sehr gute Lagerstabilitäten für die abgefüllten Referenzflüssigkeiten erzielt werden können.

Ein erster Volumenanteil im flexiblen Beutel gemäß US 6,405,872 wird von der tonometrierten Flüssigkeit beansprucht, welche zumindest eine gelöste Gaskomponente enthält. Weiters ist im flexiblen Beutel eine einen zweiten Volumenanteil beanspruchende Gasphase vorgesehen, welche zumindest die in der Flüssigkeit gelöste Gaskomponente enthält. Die Gasphase ist von der Flüssigkeit durch deren Flüssigkeitsoberfläche getrennt, die als Austauschfläche wirkt. Da das Volumen der Flüssigkeit und das Volumen der Gasphase in Summe kleiner als das maximale Füllvolumen des flexiblen Behälters ist, entspricht der Innendruck im Behälter dem atmosphärischen Außendruck, selbst dann, wenn (innerhalb vorgegebener Grenzen) Temperatur- und Druckänderungen auftreten.

Zur Entnahme der Flüssigkeit aus dem flexiblen Beutel kann gemäß EP 2 077 452 B1 direkt bei der Einmündung der jeweiligen Anschlussleitung in den Beutel ein vom Analysator ansteuerbares Mehrwegeventil mit zumindest zwei Ventilstellungen angeordnet sein, wobei die erste Ventilstellung eine Fluidverbindung zwischen der Anschlussleitung und dem flexiblen Beutel herstellt und die zweite Ventilstellung den flexiblen Beutel verschließt und eine Fluidverbindung zwischen einer Belüftungsquelle, vorzugsweise der Umgebungsluft, und der Anschlussleitung herstellt.

Aus der US 7,188,993 B1 ist ein Verfahren und eine Vorrichtung zur resonanten Vibrationsmischung von Flüssigkeiten, festen Partikeln und/oder Gasen bekannt. Die Vorrichtung weist in einem festen Gehäuse drei unabhängig voneinander bewegbare, mit Federsystemen verbundene Massen auf, wobei eine dieser Massen, die Oszillatormasse, von einem Elektromotor in Schwingung versetzt wird. Eine Mischkammer, welche die zu mischenden Substanzen aufnimmt, ist mit einer der beiden anderen Massen fest verbunden. Ein Ausführungsbeispiel beschreibt die Anwendung der Vorrichtung auf eine Mischung aus einer Flüssigkeit und einem Gas zur Herstellung von 'gasified media', in welchem das Gas in Form kleinster Gasblasen (micro sized bubbles) für längere Zeit in die Flüssigkeit eingelagert bleibt. Aufgrund der Blasenbildung in der Flüssigkeit sind derartige Verfahren zur Beschleunigung der Äquilibrierung von Referenzflüssigkeiten eines Analysators nicht geeignet.

Aus der US 2011/0065084 A1 ist ein System und ein Verfahren zur Messung und Regelung von Sauerstoff in einem starrwandigen Kulturgefäß bekannt, das Zellen in einem Kulturmedium und darüber eine Gasphase enthält. An der Innenwand des Kulturgefäßes ist ein mit dem Kulturmedium in Kontakt stehender optischer Sauerstoffsensor befestigt, der von außen mit einer Lichtquelle angeregt und von einem Fotodetektor überwacht wird, dessen Signal einer Steuereinheit zugeführt wird. Weiters ist an der Außenwand des Kulturgefäßes ein Schwingungsmischer befestigt, der basierend auf dem Feedback des Sauerstoffsensors Schwingungsenergie an das starrwandige Kulturgefäß abgibt, um den Sauerstofftransport zu erhöhen und eine rasche Gleichgewichtseinstellung zwischen dem Kulturmedium und der Gasphase herzustellen.

Aufgabe der Erfindung ist es, ein Verfahren und ein System mit einer Vorrichtung zur Beschleunigung der Äquilibrierung einer Flüssigkeit, vorzugsweise einer Referenzflüssigkeit eines Analysators, vorzuschlagen, deren Flüssigkeitsoberfläche mit einer Gasphase (beispielsweise als Reservoir für O$_2$, welches gelöst in der wässrigen Lösung nur in deutlich geringeren Konzentrationen vorhanden ist) in Kontakt steht, wobei die Flüssigkeit samt Gasphase in einem flexiblen Beutel vorliegt, und wobei ein oder mehrere Beutel vorzugsweise in einer in einen Analysator (austauschbar) einsetzbaren Kassette vorliegen.

Diese Aufgabe wird erfindungsgemäß dadurch gelöst, dass die Energie einer mechanischen Schwingung eines Geberelements in die im flexiblen Beutel vorliegende Flüssigkeit eingetragen wird, wobei die Eintragung der mechanischen Energie über zumindest einen mit dem Geberelement in Kontakt stehenden Wandbereich des flexiblen Beutels erfolgt. Durch die erfindungsgemäße Maßnahme wird die Äquilibrierungszeit der Gaswerte bzw. der davon abhängigen Werte der einzelnen Referenzflüssigkeiten wesentlich verkürzt, so dass der Messbetrieb deutlich beschleunigt werden kann.

Bevorzugt erfolgt die Eintragung mechanischer Energie in die Flüssigkeit nach dem Einsetzen einer neuen Kassette in den Analysator bzw. nach einer längeren Standzeit des Analysators und vor der jeweiligen Entnahme von Flüssigkeit aus dem flexiblen Beutel. So ist bei jeder Entnahme von Kalibrations- oder Kontrollflüssigkeit garantiert, dass die Gaswerte mit hinreichender Genauigkeit eingestellt sind. Unter einer längeren Standzeit (die seit der letzten Entnahme von Flüssigkeit aus dem jeweiligen Beutel vergangene Zeit) sind insbesondere Standzeiten von mehr als einer Stunde zu verstehen.

Alternativ erfolgt die Eintragung mechanischer Energie in die Flüssigkeit nach signifikanten Druck- oder Temperaturänderungen am Aufstellungs- oder Betriebsort des Analysators. Unter einer signifikanten Druckänderung versteht man beispielsweise die Zu- oder Abnahme des äußeren Luftdrucks um 10 mbar, vorzugsweise 20 mbar, vorzugsweise 30 mbar oder mehr. Unter einer signifikanten Temperaturänderung versteht man beispielsweise die Zu- oder Abnahme des Umgebungstemperatur am Aufstellungs- oder Betriebsort um 1°C, vorzugsweise 2 °C, vorzugsweise 3°C oder mehr.

Eine Durchführung des erfindungsgemäßen Verfahrens ist insbesondere dann vorgesehen, wie eine solche Druck- oder Temperaturänderung relativ rasch erfolgt, beispielsweise innerhalb eines Zeitraums von 12h.

Erfindungsgemäß werden bevorzugt mechanische Schwingungen in einem Frequenzbereich von Infraschall (ca. 1 Hz) bis 500 Hz, vorzugsweise von 25 bis 50 Hz, mit einer Amplitude von 0,5 bis 5 mm, vorzugsweise von 1 bis 3 mm, eingekoppelt.

Besonders gute Ergebnisse können erzielt werden, wenn die mechanische Energie in Form von trapez- oder sägezahnförmigen Schwingungen, vorzugsweise mit steilen Flanken, oder als periodische Stoßwelle eingekoppelt wird.

Insbesondere soll darauf geachtet werden, dass die Einkopplung der mechanischen Schwingung unter Vermeidung einer resonanten Anregung erfolgt.

Eine resonante Anregung des Systems erzeugt an der Phasengrenzfläche Oberflächenwellen. Diese Eigenschaft vergrößert die Phasengrenzfläche, führt aber nicht zur erwünschten Vermengung in der flüssigen Phase. Bei Resonanz wird weiter die Auslenkung des Systems so vergrößert, dass sich die flüssige Phase mit dem Gasraum vermischt. Dies führt zu ungewollten Gaseinschlüssen (Bläschen) in der flüssigen Phase.

Eine Ausführungsform des erfindungsgemäßen Systems mit einer Vorrichtung zur Beschleunigung der Äquilibrierung einer Flüssigkeit, deren Flüssigkeitsoberfläche mit einer Gasphase in Kontakt steht, zeichnet sich dadurch aus, dass die Kassette zumindest ein in eine Schwingbewegung versetzbares Geberelement aufweist, das den flexiblen Beutel in zumindest einem Wandbereich mechanisch kontaktiert.

In einer alternativen Ausführungsform des erfindungsgemäßen Systems mit einer Vorrichtung zur Beschleunigung der Äquilibrierung einer Flüssigkeit, deren Flüssigkeitsoberfläche mit einer Gasphase in Kontakt steht, kann das Geberelement auch analysatorseitig vorliegen und die Kassette weist einen Eingriffsbereich (z.B. eine entsprechende Öffnung im Kassettenboden) auf, durch welchen das analysatorseitige Geberelement mit dem in der Kassette enthaltenen flexiblen Beutel in Kontakt bringbar ist.

Die Erfindung wird im Folgenden anhand von zum Teil schematischen Darstellungen näher erläutert. Es zeigen:

Fig. 1　ein erfindungsgemäßes System mit einem Analysator und einer Vorrichtung zur Beschleunigung der Gleichgewichtseinstellung in einer Flüssigkeit, in einer dreidimensionalen Darstellung;

Fig. 2　eine Ausführungsvariante des erfindungsgemäßen Systems mit einem Analysator und einer Vorrichtung zur Beschleunigung der Gleichgewichtseinstellung einer Flüssigkeit, in einer Schnittdarstellung;

Fig. 3　eine schematische Darstellung der bei einer Gleichgewichtseinstellung zwischen Flüssigkeit und Gasphase ohne Eintrag mechanischer Energie stattfindenden Prozesse;

Fig. 4　eine schematische Darstellung der bei einer Gleichgewichtseinstellung zwischen Flüssigkeit und Gasphase mit Eintrag mechanischer Energie stattfindenden Prozesse;

Fig. 5　ein Diagramm möglicher Schwingungsformen, mit welchen ein erfindungsgemäßes Geberelement angesteuert werden kann;

Fig. 6　Ansichten einer Anordnung mit einem zum Teil gefüllten, flexiblen Beutel zu unterschiedlichen Zeitpunkten, in Ruhe (d.h. ohne zusätzlichen Eintrag mechanischer Energie);

Fig. 7     Ansichten einer Anordnung mit einem zum Teil gefüllten, flexiblen Beutel zu unterschiedlichen Zeitpunkten, mit Eintrag von mechanischer Energie;

Fig. 8     ein Diagramm einer Gleichgewichtseinstellung von $O_2$ einer Flüssigkeit. Die drei Kurven zeigen ein System aus drei Beuteln, wie es in Fig. 2 ersichtlich ist. Dreieck: erster, an das Geberelement angekoppelter, unterer Beutel. Rechteck: mittlerer Beutel. Kreis: oberster Beutel;

Fig. 9     ein Diagramm einer $O_2$-Gleichgewichtseinstellung eines "liegenden" Beutels (10° Schrägstellung) mit (Fig. 9b) und ohne (9a) erfindungsgemäßer Einkopplung von mechanischer Energie; sowie

Fig. 10     ein Diagramm einer $O_2$-Gleichgewichtseinstellung eines "stehenden" Beutels (80° Schrägstellung) mit (Fig. 10b) und ohne (10a) erfindungsgemäßer Einkopplung von mechanischer Energie.

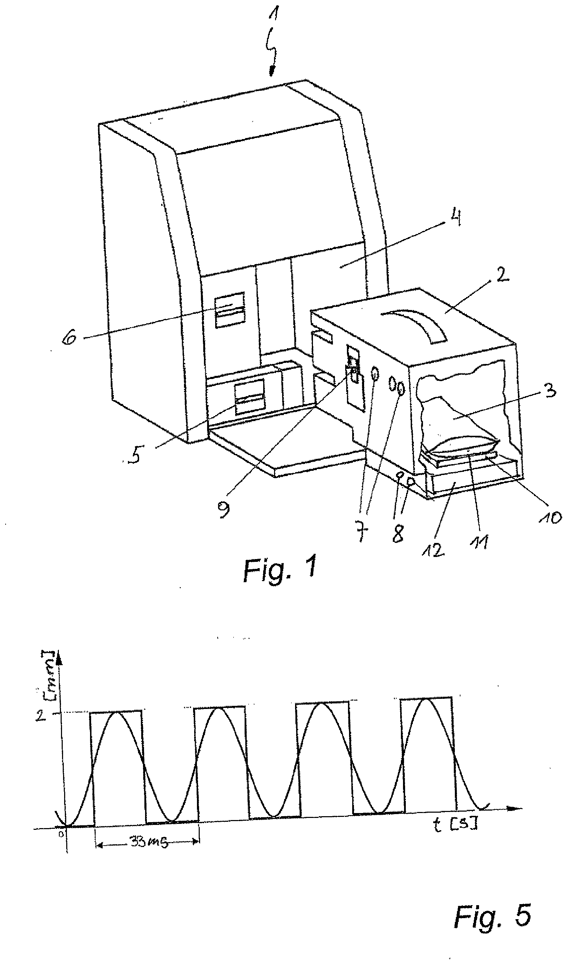

Fig. 1 zeigt in schematischer Darstellung einen Analysator 1, beispielsweise einen Blutgas-Analysator, bei welchem die für den laufenden Betrieb benötigten Betriebs- und Verbrauchsmaterialien in Form von Einweg-Kassetten und/oder Modulen in den Analysator einsetzbar sind.

Die Kassette 2 (Fluidpack), die in eine Aufnahme 4 des Analysators 1 einsetzbar ist, enthält Flüssigkeits- und Abfallbehälter, insbesondere flexible gasdichte Beutel 3, welche die zum Betrieb des Analysators 1 benötigten Referenzflüssigkeiten, insbesondere Kontrollflüssigkeiten für die Qualitätskontrolle und Kalibrationsflüssigkeiten zur Kalibration der Sensoren enthalten.

Optional können die Kontrollflüssigkeiten für die Qualitätskontrolle auch in einer separaten Kassette 5 des Analysators 1 angeordnet sein und bei Bedarf durch den Anwender unabhängig von der Kassette mit den restlichen Referenz- und Betriebsflüssigkeiten auf einfache Weise ausgewechselt werden.

Weiters weist der Analysator 1 eine Sensorkassette 6 auf, die zumindest einen Teil der für die Analytbestimmung benötigten Sensoren enthält.

Die Kassette 2 enthält auch die hier nicht dargestellten Anschlussleitungen zu den flexiblen Beuteln 3, die jeweils zu einem Andocknippel 7 an der Außenseite der Kassette führen, so dass nach dem Einsetzen der Kassette 3 in die Aufnahme 4 des Analysators 1 automatisch eine fluiddichte Verbindung zu den entsprechenden Anschlussstellen des Analysators hergestellt werden kann. Weiters können auch elektrische Kontakte 8 an der Außenseite der Kassette 2 vorgesehen sein, die entsprechenden elektrischen Kontakten in der Aufnahme 4 zugeordnet sind.

An der Kassette 2 ist ein Speichermodul 9 befestigt, beispielsweise ein Speicherchip oder ein RFID-Chip, welcher beispielsweise Informationen über die - Referenzflüssigkeiten in der Kassette und deren jeweilige Zielwerte enthält, die vom Analysator 1 nach dem Einsetzen der Kassette 2 automatisch ausgelesen werden.

Zur Einbringung mechanischer Schwingungen in den flexiblen Beutel 3 weist die Kassette 2 gemäß Fig. 1 zumindest ein in eine Schwingbewegung versetzbares Aktorelement bzw. Geberelement 10 auf, das zumindest einen flexiblen Beutel 3 in zumindest einem Wandbereich 11 mechanisch kontaktiert.

Das Geberelement 10 steht mit einer Antriebsvorrichtung 12 in Verbindung, die entweder - wie in Fig. 1 dargestellt - in der Kassette 2 angeordnet ist oder besonders bevorzugt auch als Teil des Analysators 1 ausgeführt sein kann und beim Kassettenwechsel im Analysator 1 verbleibt.

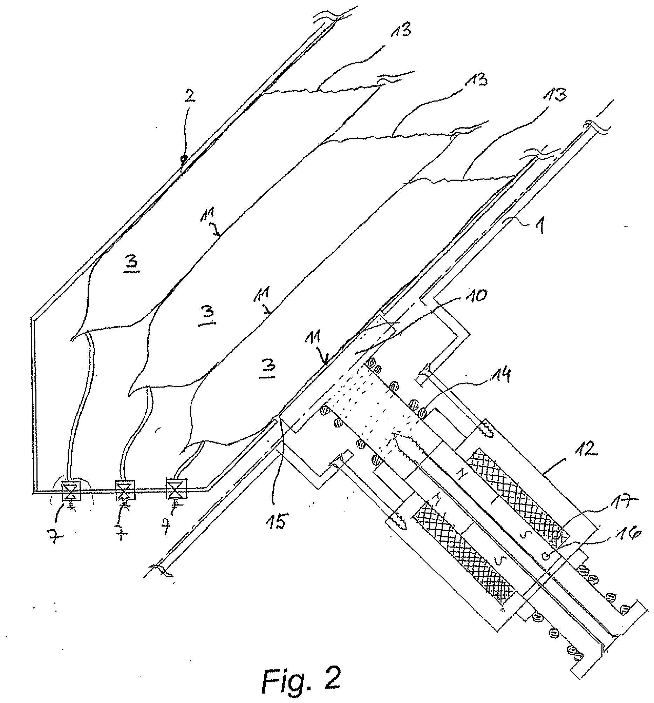

Bei der in Fig. 2 dargestellten Ausführungsvariante einer austauschbaren Kassette 2 sind mehrere flexible Beutel 3 gestapelt angeordnet, wobei deren Wandbereiche 11 zur Übertragung der Schwingbewegung direkt oder indirekt miteinander in Kontakt stehen. Dabei pflanzt sich die Energie der vom Geberelement 10 erzeugten mechanischen Schwingung durch den ersten, zuunterst liegenden Beutel 3 in die anliegenden weiteren Beutel 3 fort. Die Flüssigkeitsoberfläche in den einzelnen flexiblen Beuteln 3 ist mit 13 bezeichnet. Die Beutel 3 sind an deren oberen Enden in der Kassette 2 befestigt (nicht dargestellt), um nicht nach unten abzurutschen.

Die Beutel 3 können auch indirekt in Verbindung stehen, indem beispielsweise Zwischenlagen oder Trennungsstege zwischen den Beuteln 3 eingeschoben sind oder die Beutel auf einem gemeinsamen Träger aufliegen, beispielsweise einem regalförmigen Haltegestell. In letzterem Fall kann die Einkopplung der mechanischen Energie über den gemeinsamen Träger erfolgen, so dass dieser auch die Funktion des Geberelements 10 erfüllt.

In Fig. 2 ist eine zu Fig. 1 alternative Ausgestaltung der Kassette 2 dargestellt, in welcher das Geberelement 10 nicht Bestandteil der Kassette 2 ist, sondern als ein Teil des Analysators 1 ausgeführt ist und im dargestellten Beispiel über eine federgelagerte Aufnahme 14 mit der ebenfalls analysatorseitig angeordneten Antriebsvorrichtung 12 (mit Permanentmagnet 16 und Magnetspule 17) in Verbindung steht. In dieser Ausführungsform weist die Kassette 2 an ihrer Unterseite eine entsprechende Ausnahme oder Öffnung 15 auf, durch welche im Betriebszustand das analysatorseitige Geberelement 10 in den Innenraum der Kassette 2 eingebracht werden kann und so den hier angrenzenden flexiblen Beutel 3 in zumindest einem Wandbereich 11 mechanisch kontaktiert.

Besonders bevorzugt sind flexible Beutel 3 aus zwei im Wesentlichen rechteckförmigen Wandelementen, die an ihren Rändern miteinander verschweißt sind (sogenannte envelope-förmige Beutel).

Hochgestellte (envelope-förmige) flexible Beutel weisen eine besonders hohe und schmale Flüssigkeitssäule mit verhältnismäßig kleiner Flüssigkeitsoberfläche und langen Diffusionsstrecken auf. Vorteile dieser Anordnung sind, dass die Flüssigkeit gut entnommen werden kann, die Einstellzeiten (Zeit bis zur Gleichgewichtseinstellung) sind jedoch länger.

Liegende (envelope-förmige) flexible Beutel weisen eine besonders niedrige und breite Flüssigkeitssäule mit verhältnismäßig großer Flüssigkeitsoberfläche und kurzen Diffusionsstrecken auf. Vorteile dieser Anordnung sind, dass die Einstellzeiten (Zeit bis zur Gleichgewichtseinstellung) kurz sind, die Flüssigkeit aber umständlicher und evtl. nicht vollständig entnommen werden kann.

Besonders bevorzugt sind flexible Beutel 3, die schräg liegend, mit einem Neigungswinkel zur Horizontalebene von 5° bis 80°, vorzugsweise 10° bis 50°, in der Kassette 2 gelagert sind (Fig. 2).

Bevorzugt kann dabei die Kontaktfläche des Geberelementes 10 20% bis 70%, vorzugsweise 25% bis 50%, der Auflagefläche 11 des flexiblen Beutels 3 in der Kassette 2 betragen.

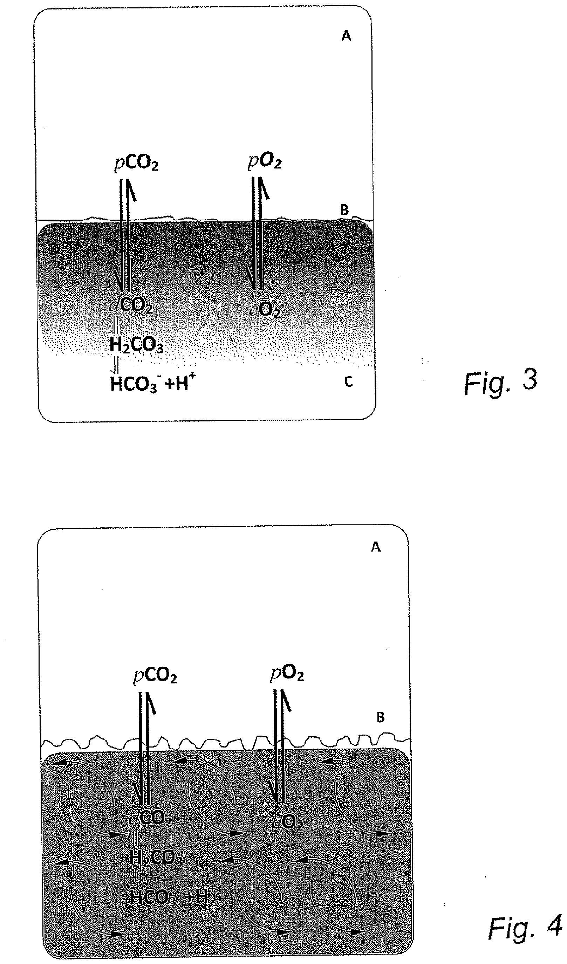

Der erfindungsgemäße Lösungsansatz verfolgt die in den Fig. 3 und 4 skizzierte Idee der erzwungenen Umwälzung der im flexiblen Beutel 3 vorliegenden flüssigen Phase und der Vergrößerung der Phasengrenzfläche zwischen Flüssigkeit und Gasphase durch mechanischen Energieeintrag (Vibration). Fig. 3 zeigt die Situation ohne und Fig. 4 mit mechanischem Energieeintrag, wobei die verwendeten Zeichen folgende Bedeutung haben:

A        Gasphase (head space)
B        Phasengrenzfläche (interphase)
C        wässrige Lösung
$pCO_2$     Partialdruck von Kohlendioxid in der Gasphase
$pO_2$      Partialdruck von Sauerstoff in der Gasphase
$cCO_2$     Konzentration von gelöstem Kohlendioxid
$cO_2$      Konzentration von gelöstem Sauerstoff

Es zeigt sich eine erhebliche Verkürzung der Gleichgewichtseinstellung der Blutgasparameter ($pO_2$, $pCO_2$, pH). Diese Verkürzung der Einstellzeit ist so signifikant, dass sich bereits nach maximal ca. 60 s keine Differenz zu den Sollwerten einstellt. Es wurden Untersuchungen angestellt, unter welchen Bedingungen eine rasche Einstellung des Gleichgewichts in einem System aus einer Flüssigkeit und einer Gasphase eintritt. Die Vibrationseinrichtung (Fig. 2) übt eine lineare Bewegung aus, da mit dieser die bestmöglichen Ergebnisse erzielt werden. Als Geberelement 10 kann beispielsweise eine Polycarbonatplatte eingesetzt werden, die an der federgelagerten Aufnahme 14 befestigt ist. Die Magnetspule 17 der analysatorseitigen Antriebsvorrichtung 12 wird von einem Funktionsgenerator über einen Leistungsoperationsverstärker betrieben.

Wie aus Fig. 5 ersichtlich können erfindungsgemäß Vibrationen umfassend rein sinusförmige bis rechteckförmige Vibrationsbewegungen mit steilen Flanken verwendet werden.

Mögliche Antriebsvorrichtungen sind beispielsweise Schwingungsaktoren wie piezoelektrische Aktoren, insbesondere Multilayer-Piezoaktoren, Motoren, insbesondere Exzenter-Motoren, Motoren mit Pleuelkopplung oder auch vorzugsweise elektromagnetische Schwingungssysteme, insbesondere solche mit Feder-Masse-Feder Systemen.

Als vorteilhaft haben sich Schwingungen in einem Frequenzbereich von 1 bis 500 Hz, vorzugsweise von 25 bis 50 Hz, mit einer Amplitude von 0,5 bis 5 mm, vorzugsweise von 1 bis 3 mm erwiesen.

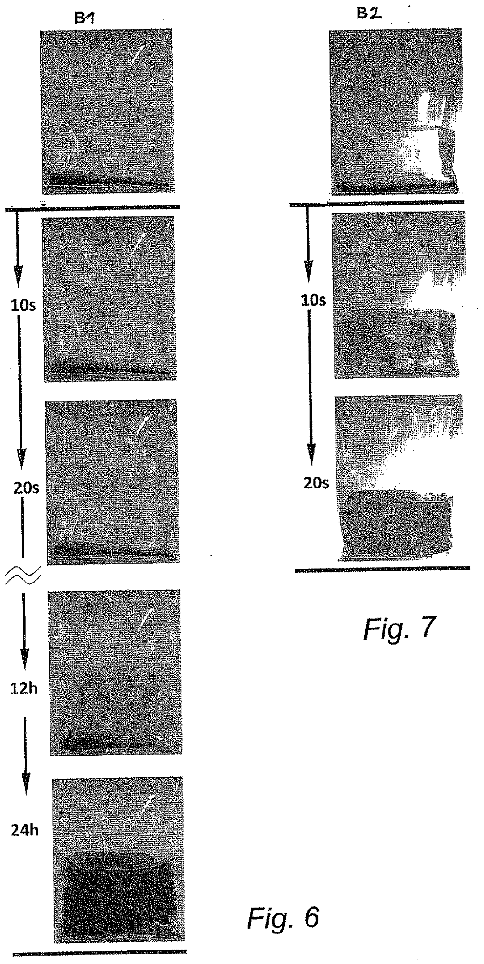

Mit einer in den Fig. 6 und 7 dargestellten Versuchsanordnung, bei welcher aufrecht stehende, envelope-förmige, flexible Beutel mit jeweils 100 ml Flüssigkeit und 100 ml Gasphase verwendet wurden, kann die verbesserte Dynamik bei der Einstellung einer gleichmäßigen Verteilung eines Stoffes innerhalb einer Flüssigkeitsphase deutlich gemacht werden. Die flexiblen Laminatbeutel B1 und B2 wurden mit 100 ml pH-gepufferter Kochsalzlösung und 100 ml Luft befüllt. Im Anschluss wurden die Lösungen jeweils mit 5ml Farbstofflösung durch Injektion unterschichtet. Beutel B1 bleibt in Ruhe und simuliert mittels der Farbstoffverteilung den zeitlichen Verlauf der Diffusion eines Stoffes innerhalb der Lösung. Beutel B2 wird mit der in Fig. 2 beschriebenen Versuchseinrichtung gerüttelt.

Im Beutel B1 (siehe Fig. 6) konnte erst nach 24h kein Farbgradient mehr ausgemacht werden, wohingegen im Beutel B2 (siehe Fig. 7) die homogene Vermengung bereits nach 20s abgeschlossen ist. Dieser Modellversuch zeigt, dass durch den erfindungsgemäßen Eintrag von mechanischer Energie in den Beutel die Durchmischung innerhalb der Flüssigkeitsphase deutlich beschleunigt werden kann.

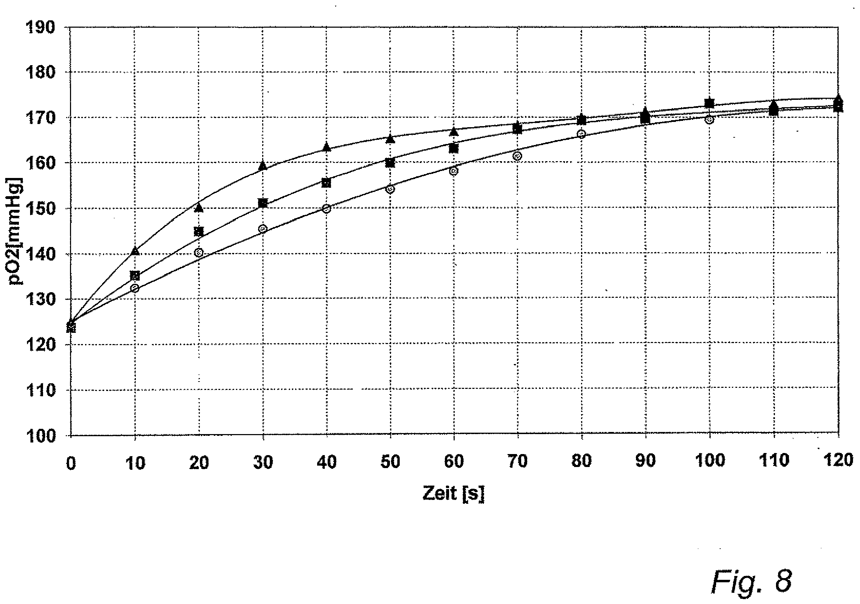

Fig. 8 zeigt die Verläufe der Gleichgewichtseinstellung von $O_2$ eines unter Fig. 2 beschriebenen Systems mit drei übereinanderliegenden, flexiblen Beuteln 3.

Dabei wurden 100 ml einer Gasphase (Luft mit 170 mmHg $O_2$) in den Headspace-freien Beutel, der 100 ml einer mit 120 mmHg $O_2$ äquilibrierten Salzlösung enthielt, durch Injektion bei Raumtemperatur (ca. 22 °C) mittels Spritze als Headspace eingebracht. Mechanische Energie wurde über eine Vorrichtung analog Fig. 2 bei einer Frequenz von 32 Hz und 1,5 mm Schwingungsamplitude eingekoppelt. Es wurden in definierten Abständen Proben über eine im untersten Punkt des Beutels angeschlossene Leitung entnommen und der $pO_2$-Wert mittels eines Blutgasanalysators bestimmt.

Es zeigt, dass der zuunterst liegende und sich direkt mit dem Geberelement 10 in Kontakt befindende Beutel (Dreiecke) die rascheste Äquilibrierung erfährt, aber auch die darüber liegenden Beutel (Quadrate bzw. Kreise) nur unwesentlich langsamer äquilibriert werden und nach ca. 90 Sekunden bereits alle drei Beutel im Wesentlichen gleichmäßig äquilibriert vorliegen.

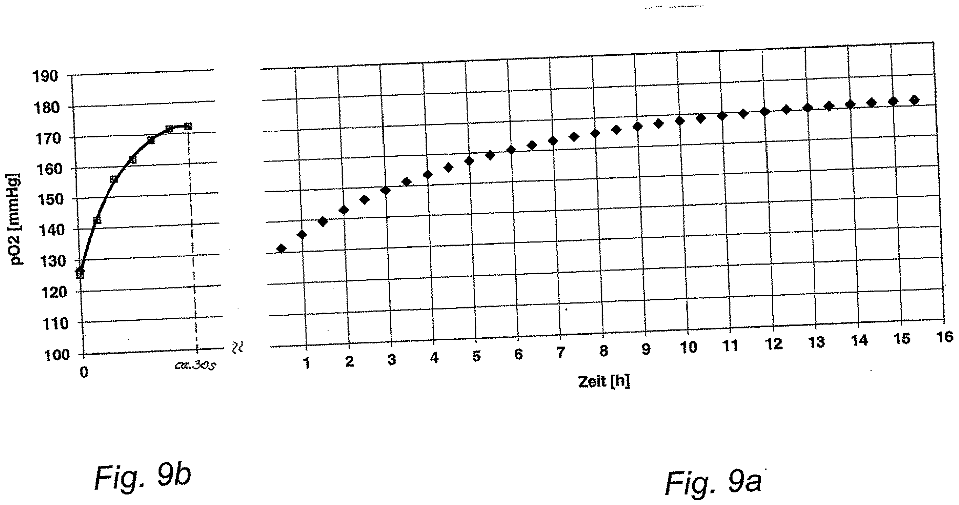

Fig. 9 zeigt die Gleichgewichtseinstellung eines liegenden Beutels (ca. 10° Schräglage zur Horizontalen) von $O_2$ mit und ohne erfindungsgemäße Einkopplung von Energie. Dabei wurden 100 ml einer Gasphase (Luft mit 170 mmHg $O_2$) in den Headspace-freien Beutel, der 100 ml einer mit 120 mmHg $O_2$ äquilibrierten Salzlösung enthielt, durch Injektion bei Raumtemperatur (ca. 22 °C) mittels Spritze als Headspace eingebracht. Anschließend wurden in definierten Abständen Proben über eine im untersten Punkt des Beutels angeschlossene Leitung entnommen und der $pO_2$-Wert mittels eines Blutgasanalysators bestimmt. Die Zeit bis zur Gleichgewichtseinstellung beträgt ca. 15h (Fig. 9a). Danach wurde der Versuch wiederholt, wobei Energie über eine Vorrichtung nach Fig. 2 bei einer Frequenz von 32 Hz und 1,5 mm Schwingungsamplitude eingekoppelt wurde. Die Zeit bis zur Gleichgewichtseinstellung beträgt nur ca. 30 Sek. (Fig. 9b). Für die in Fig. 9a gezeigten Werte ohne Einkopplung einer mechanischen Energie wurde eine $t_{50}$-Zeit (Zeit, bis 50% des Endwertes der Äquilibrierung erreicht sind) von 5 h und eine $t_{70}$-Zeit (Zeit, bis 70% des Endwertes der Äquilibrierung erreicht sind) von 7 h ermittelt. Für die in Fig. 9b gezeigten Werte mit Einkopplung einer mechanischen Energie wurde hingegen eine deutlich verringerte $t_{50}$-Zeit von 10 sec und eine $t_{70}$-Zeit von 14 sec ermittelt.

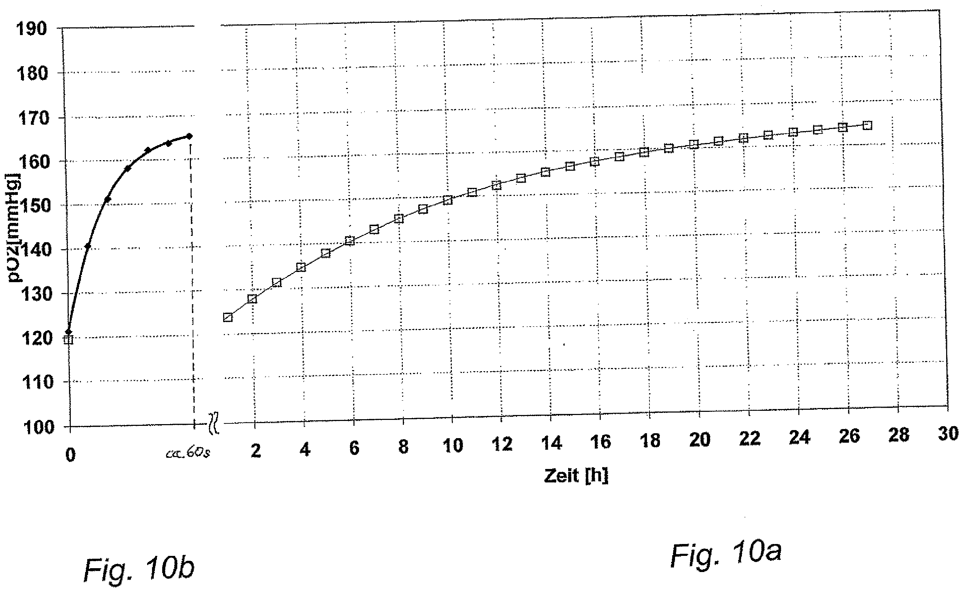

Fig. 10 zeigt die Gleichgewichtseinstellung eines stehenden Beutels (ca. 80° Schräglage zur Horizontalen) von $O_2$ mit und ohne erfindungsgemäße Einkopplung von Energie. Dabei wurden 100 ml einer Gasphase (Luft mit 170 mmHg $O_2$) in den Headspace-freien Beutel, der 100 ml einer mit 120 mmHg $O_2$ äquilibrierte Salzlösung enthielt, durch Injektion bei Raumtemperatur (ca. 22 °C) mittels Spritze als Headspace eingebracht. Anschließend wurden in definierten Abständen Proben über eine im untersten Punkt des Beutels angeschlossene Leitung entnommen und der $pO_2$-Wert mittels eines Blutgasanalysators bestimmt. Die Zeit bis zur Gleichgewichtseinstellung beträgt ca. 26h (Fig.10a). Danach wurde der Versuch wiederholt, wobei Energie über eine Vorrichtung nach Fig. 2 bei einer Frequenz von 32 Hz und 1,5 mm Schwingungsamplitude eingekoppelt wurde. Die Zeit bis zur Gleichgewichtseinstellung beträgt ca. 60 Sek.(Fig. 10b). Für die in Fig. 10a gezeigten Werte ohne Einkopplung einer mechanischen Energie wurde eine $t_{50}$-Zeit von 7 h und eine $t_{70}$-Zeit von 13 h ermittelt. Für die in Fig. 10b gezeigten Werte mit Einkopplung einer mechanischen Energie wurde hingegen eine deutlich verringerte $t_{50}$-Zeit von 15 sec und eine $t_{70}$-Zeit von 24 sec ermittelt.

Die Betriebstemperatur der Flüssigkeit im Beutel liegt üblicherweise bei Raumtemperatur oder leicht darüber.

**Patentansprüche**

1. Verfahren zur Beschleunigung der Äquilibrierung einer Flüssigkeit, vorzugsweise einer Referenzflüssigkeit eines Analysators, deren Flüssigkeitsoberfläche mit einer Gasphase in Kontakt steht, wobei die Flüssigkeit und die Gasphase in einem flexiblen, gasdichten Beutel (3) einer in einen Analysator (1) austauschbar einsetzbaren Kassette (2) aufgenommen sind, **dadurch gekennzeichnet, dass** die Energie einer mechanischen Schwingung eines Geberelements (10) in die im flexiblen Beutel (3) vorliegende Flüssigkeit eingetragen wird, wobei die Eintragung der mechanischen Energie über zumindest einen mit dem Geberelement (10) in Kontakt stehenden Wandbereich (11) des flexiblen Beutels (3) erfolgt.

2. Verfahren nach Anspruch 1, **dadurch gekennzeichnet, dass** die Eintragung mechanischer Energie in die Flüssigkeit zumindest nach dem Einsetzen einer neuen Kassette (2) in den Analysator (1) und vor der erstmaligen Entnahme von Flüssigkeit aus dem flexiblen Beutel (3) erfolgt.

3. Verfahren nach Anspruch 1, **dadurch gekennzeichnet, dass** die Eintragung mechanischer Energie in die Flüssigkeit nach längerer Standzeit des Analysators (1) und vor der Entnahme von Flüssigkeit aus dem flexiblen Beutel (3) erfolgt.

4. Verfahren nach Anspruch 1, **dadurch gekennzeichnet, dass** die Eintragung mechanischer Energie in die Flüssigkeit nach signifikanten Druck- oder Temperaturänderungen am Aufstellungsort des Analysators (1) erfolgt.

5. Verfahren nach einem der Ansprüche 1 bis 4, **dadurch gekennzeichnet, dass** die Kassette (2) mehrere flexible Beutel (3) enthält und die Eintragung mechanischer Energie in die Flüssigkeiten benachbarter flexibler Beutel (3) über aneinander liegende Wandbereiche (11) dieser flexiblen Beutel (3) erfolgt.

6. Verfahren nach einem der Ansprüche 1 bis 5, **dadurch gekennzeichnet, dass** die mechanische Energie in Form von periodischen Stoßwellen eingetragen wird.

7. Verfahren nach einem der Ansprüche 1 bis 5, **dadurch gekennzeichnet, dass** mechanische Energie in einem Frequenzbereich von 1 bis 500 Hz, vorzugsweise von 25 bis 50 Hz, mit einer Amplitude von 0,5 bis 5 mm, vorzugsweise von 1 bis 3 mm, eingekoppelt wird.

8. Verfahren nach einem der Ansprüche 1 bis 5, **dadurch gekennzeichnet, dass** mechanische Energie in Form von trapez- oder sägezahnförmigen Schwingungen, bevorzugt mit steilen Flanken, eingekoppelt wird.

9. Verfahren nach einem der Ansprüche 1 bis 8, **da-**

**durch gekennzeichnet, dass** die Eintragung der mechanischen Schwingung unter Vermeidung einer resonanten Anregung erfolgt.

10. System, welches einen Analysator (1) sowie eine in den Analysator (1) austauschbar einsetzbare Kassette (2) umfasst, die zumindest einen flexiblen, gasdichten Beutel (3) beinhaltet, welcher eine Flüssigkeit, vorzugsweise eine Referenzflüssigkeit des Analysators (1), enhält, **dadurch gekennzeichnet, dass** das System eine Vorrichtung zur Beschleunigung der Äquilibrierung der Flüssigkeit aufweist, deren Flüssigkeitsoberfläche mit einer Gasphase in Kontakt steht, wobei die Kassette (2) oder der Analysator (1) zumindest ein in eine Schwingbewegung versetzbares Geberelement (10) aufweist, das den flexiblen Beutel (3) zur Eintragung mechanischer Energie in die Flüssigkeit in zumindest einem Wandbereich (11) mechanisch kontaktiert.

11. System nach Anspruch 10, **dadurch gekennzeichnet, dass** in der austauschbaren Kassette (2) mehrere flexible Beutel (3) angeordnet sind, deren Wandbereiche (11) zur Übertragung der Schwingbewegung direkt oder indirekt miteinander in Kontakt stehen.

12. System nach Anspruch 10, **dadurch gekennzeichnet, dass** in der austauschbaren Kassette (2) mehrere flexible Beutel (3) angeordnet sind, deren Wandbereiche (11) zur Übertragung der Schwingbewegung jeweils einem separaten Geberelement (10) zugeordnet sind.

13. System nach einem der Ansprüche 10 bis 12, **dadurch gekennzeichnet, dass** das Geberelement (10) mit einer Antriebsvorrichtung (12) in Verbindung steht.

14. System nach einem der Ansprüche 10 bis 13, **dadurch gekennzeichnet, dass** jeder flexible Beutel (3) aus zwei im Wesentlichen rechteckförmigen Wandelementen besteht, die am Rand verschweißt sind.

15. System nach einem der Ansprüche 10 bis 14, **dadurch gekennzeichnet, dass** jeder flexible Beutel (3) schräg liegend, mit einem Neigungswinkel zur Horizontalebene von 5° bis 80°, vorzugsweise 10° bis 50°, in der Kassette (2) gelagert ist.

16. System nach einem der Ansprüche 10 bis 15, **dadurch gekennzeichnet, dass** die Kontaktfläche des Geberelementes (10) 20% bis 70%, bevorzugt 25% bis 50%, der Auflagefläche des flexiblen Beutels (3) in der Kassette (2) beträgt.

**Claims**

1. Method for accelerating the equilibration of a liquid, preferably a reference liquid of an analyzer, whose liquid surface is in contact with a gas phase, wherein the liquid and the gas phase are accommodated in a flexible, gas-tight bag (3) of a cartridge (2) which can be used interchangeably in an analyzer (1), **characterised in that** the energy of a mechanical oscillation of an actuator element (10) is introduced into the liquid present in the flexible bag (3), wherein the introduction of the mechanical energy takes place via at least one wall area (11) of the flexible bag (3) which is in contact with the actuator element (10).

2. Method according to claim 1, **characterised in that** the introduction of mechanical energy into the liquid takes place at least after the insertion of a new cartridge (2) into the analyzer (1) and before the first removal of liquid from the flexible bag (3).

3. Method according to claim 1, **characterised in that** the introduction of mechanical energy into the liquid takes place after a longer idle period of the analyzer (1) and before the removal of liquid from the flexible bag (3).

4. Method according to claim 1, **characterised in that** the introduction of mechanical energy into the liquid takes place after significant pressure or temperature changes at the installation location of the analyzer (1).

5. Method according to one of the claims 1 to 4, **characterised in that** the cartridge (2) contains a plurality of flexible bags (3) and the introduction of mechanical energy into the liquids of adjacent flexible bags (3) takes place via adjacent wall areas (11) of said flexible bags (3).

6. Method according to one of the claims 1 to 5, **characterised in that** the mechanical energy is entered in the form of periodic shock waves.

7. Method according to one of the claims 1 to 5, **characterised in that** mechanical energy is introduced in a frequency range of 1 to 500 Hz, preferably from 25 to 50 Hz, with an amplitude of 0.5 to 5 mm, preferably from 1 to 3 mm.

8. Method according to one of the claims 1 to 5, **characterised in that** mechanical energy is introduced in the form of trapezoidal or sawtooth-shaped vibrations, preferably with steep flanks.

9. Method according to one of the claims 1 to 8, **characterised in that** the introduction of the mechanical vibration takes place while avoiding a resonant excitation.

10. System, comprising an analyzer (1) and a cartridge (2) which can be inserted interchangeably into the analyzer (1) and which contains at least one flexible, gas-tight bag (3) containing a liquid, preferably a reference liquid of the analyzer (1), **characterised in that** the system comprises a device for accelerating the equilibration of the liquid whose liquid surface is in contact with a gas phase, wherein the cartridge (2) or the analyzer (1) has at least one actuator element (10) which can brought to an oscillating movement and which mechanically contacts the flexible bag (3) for the introduction of mechanical energy into the liquid in at least one wall area (11).

11. System according to claim 10, **characterised in that** in the interchangeable cartridge (2) a plurality of flexible bags (3) are arranged, whose wall portions (11) are directly or indirectly in contact with each other for transmitting the oscillating motion.

12. System according to claim 10, **characterised in that** in the interchangeable cartridge (2) a plurality of flexible bags (3) are arranged, whose wall portions (11) are each associated with a separate actuator element (10) for transmitting the oscillating motion.

13. System according to one of the claims 10 to 12, **characterised in that** the actuator element (10) is in connection with a drive device (12).

14. System according to one of the claims 10 to 13, **characterised in that** each flexible bag (3) consists of two substantially rectangular wall elements which are welded at the edge.

15. System according to one of the claims 10 to 14, **characterised in that** each flexible bag (3) is mounted obliquely, with an inclination angle to the horizontal plane of 5° to 80°, preferably 10° to 50°, in the cartridge (2).

16. System according to one of the claims 10 to 15, **characterised in that** the contact surface of the actuator element (10) is 20% to 70%, preferably 25% to 50%, of the contact surface of the flexible bag (3) in the cartridge (2).

**Revendications**

1. Procédé d'accélération de l'équilibrage d'un liquide, de préférence d'un liquide de référence d'un analyseur, dont la surface de liquide est en contact avec une phase gazeuse, le liquide et la phase gazeuse étant logés dans un sachet (3) flexible, étanche aux gaz, d'une cassette (2) insérable dans un analyseur (1) de manière interchangeable, **caractérisé en ce que** l'énergie d'une oscillation mécanique d'un élément transmetteur (10) est apportée dans le liquide présent dans le sachet (3) flexible, l'apport de l'énergie mécanique étant réalisé par l'intermédiaire d'au moins une zone de paroi (11) du sachet (3) flexible en contact avec l'élément transmetteur (10).

2. Procédé selon la revendication 1, **caractérisé en ce que** l'apport d'énergie mécanique dans le liquide est réalisé au moins après l'insertion d'une nouvelle cassette (2) dans l'analyseur (1) et avant le premier prélèvement de liquide hors du sachet (3) flexible.

3. Procédé selon la revendication 1, **caractérisé en ce que** l'apport d'énergie mécanique dans le liquide est réalisé après une longue durée de service de l'analyseur (1) et avant le prélèvement de liquide hors du sachet (3) flexible.

4. Procédé selon la revendication 1, **caractérisé en ce que** l'apport d'énergie mécanique dans le liquide est réalisé après des modifications de pression ou de température significatives à l'emplacement de l'analyseur (1).

5. Procédé selon l'une quelconque des revendications 1 à 4, **caractérisé en ce que** la cassette (2) contient plusieurs sachets (3) flexibles et **en ce que** l'apport d'énergie mécanique dans les liquides de sachets (3) flexibles voisins est réalisé par l'intermédiaire de zones de paroi (11) adjacentes les unes aux autres de ces sachets (3) flexibles.

6. Procédé selon l'une quelconque des revendications 1 à 5, **caractérisé en ce que** l'énergie mécanique est apportée sous forme d'ondes de choc périodiques.

7. Procédé selon l'une quelconque des revendications 1 à 5, **caractérisé en ce que** l'énergie mécanique est couplée dans une plage de fréquence de 1 à 500 Hz, de préférence de 25 à 50 Hz, avec une amplitude de 0,5 à 5 mm, de préférence de 1 à 3 mm.

8. Procédé selon l'une quelconque des revendications 1 à 5, **caractérisé en ce que** l'énergie mécanique est couplée sous forme d'oscillations en forme de trapèze ou de dents de scie, de préférence avec des flancs raides.

9. Procédé selon l'une quelconque des revendications 1 à 8, **caractérisé en ce que** l'apport de l'oscillation mécanique est réalisé en évitant une excitation résonante.

10. Système lequel comprend un analyseur (1) ainsi qu'une cassette (2) insérable dans l'analyseur (1) de manière interchangeable, laquelle contient au moins un sachet (3) flexible, étanche aux gaz, lequel contient un liquide, de préférence un liquide de référence de l'analyseur (1), **caractérisé en ce que** le système présente un dispositif d'accélération de l'équilibrage du liquide, dont la surface de liquide est en contact avec une phase gazeuse, la cassette (2) ou l'analyseur (1) présentant au moins un élément transmetteur (10) pouvant être mis en un mouvement oscillant, lequel met en contact mécaniquement le sachet (3) flexible pour l'apport d'énergie mécanique dans le liquide dans au moins une zone de paroi (11).

11. Système selon la revendication 10, **caractérisé en ce que** plusieurs sachets (3) flexibles sont disposés dans la cassette (2) interchangeable, dont les zones de paroi (11) sont directement ou indirectement en contact entre elles pour le transfert du mouvement oscillant.

12. Système selon la revendication 10, **caractérisé en ce que** plusieurs sachets (3) flexibles sont disposés dans la cassette (2) interchangeable, dont les zones de paroi (11) sont respectivement associées à un élément transmetteur (10) séparé pour le transfert du mouvement oscillant.

13. Système selon l'une quelconque des revendications 10 à 12, **caractérisé en ce que** l'élément transmetteur (10) est en liaison avec un dispositif d'entraînement (12).

14. Système selon l'une quelconque des revendications 10 à 13, **caractérisé en ce que** chaque sachet (3) flexible est constitué de deux éléments de paroi essentiellement rectangulaires, lesquels sont soudés sur le bord.

15. Système selon l'une quelconque des revendications 10 à 14, **caractérisé en ce que** chaque sachet (3) flexible est logé dans la cassette (2) en étant logé de manière oblique, avec un angle d'inclinaison par rapport au plan horizontal de 5° à 80°, de préférence de 10° à 50°.

16. Système selon l'une quelconque des revendications 10 à 15, **caractérisé en ce que** la surface de contact de l'élément transmetteur (10) est de 20% à 70%, de préférence de 25% à 50% de la surface d'appui du sachet flexible (3) dans la cassette (2).

Fig. 1

Fig. 5

Fig. 2

*Fig. 3*

*Fig. 4*

EP 2 669 677 B1

Fig. 7

Fig. 6

16

Fig. 8

EP 2 669 677 B1

Fig. 9b

Fig. 9a

EP 2 669 677 B1

Fig. 10b

Fig. 10a

EP 2 669 677 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4116336 A, Radiometer
- US 3681255 A, Wilfore
- US 4871439 A, Enzer
- US 5780302 A, Chiron
- US 7378006 B
- US 6405872 B, Roche
- EP 2077452 B1
- US 7188993 B1
- US 20110065084 A1